# EUROPEAN PATENT SPECIFICATION

(11) **EP 4 323 764 B1**
(45) Date of publication and mention of the grant of the patent: **21.05.2025**
(21) Application number: 22721300.6
(22) Date of filing: 06.04.2022
(51) Int. Cl.: G01N 33/00

(54) **METHOD FOR MONITORING THE USE OF A METHANE INHIBITOR**
VERFAHREN ZUR ÜBERWACHUNG DER ANWENDUNG EINES METHAN INHIBITORS
PROCÉDÉ DE SURVEILLANCE DE L'UTILISATION D'UN INHIBITEUR DE MÉTHANE

(30) Priority: 13.04.2021 EP 21168204
(43) Date of publication of application: 21.02.2024
(73) Proprietor: DSM IP Assets B.V., 6221 BE Maastricht (NL)
(72) Inventor: CELI, Pietro, 4303 Kaiseraugst (CH); COVINGTON, James, Coventry CV47AL (GB); WALKER, Nicola, 4303 Kaiseraugst (CH)
(74) Representative: dsm-firmenich IP
(86) International application number: PCT/EP2022/059151
(87) International publication number: WO 2022/218780

(56) References cited:
- WO-A1-94/08738
- US-A- 3 660 562
- US-A1- 2011 192 213
- LOPES J C ET AL: "Effect of 3-nitrooxypropanol on methane and hydrogen emissions, methane isotopic signature, and ruminal fermentation in dairy cows", JOURNAL OF DAIRY SCIENCE, vol. 99, no. 7, 13 April 2016 (2016-04-13), pages 5335 - 5344, XP029602001, ISSN: 0022-0302, DOI: 10.3168/JDS.2015-10832
- PATRA AMLAN K.: "Recent Advances in Measurement and Dietary Mitigation of Enteric Methane Emissions in Ruminants", FRONTIERS IN VETERINARY SCIENCE, vol. 3, 20 May 2016 (2016-05-20), XP055837359, DOI: 10.3389/fvets.2016.00039
- PLACE SARA E. ET AL: "Construction and Operation of a Ventilated Hood System for Measuring Greenhouse Gas and Volatile Organic Compound Emissions from Cattle", ANIMALS, vol. 1, no. 4, December 2011 (2011-12-01), pages 433 - 446, XP055838059, ISSN: 2076-2615, DOI: 10.3390/ani1040433

## Description

The present invention relates to a method of increasing the volatile organic compound (VOC) emissions in a ruminant, said method comprising the administration of a methane inhibitor to said ruminant. The increase in the VOC emissions can be used for monitoring and/or quantifying the reduction of methane emanating from the digestive activities of the ruminant affected by the feeding of said methane inhibitor.

In recent years, with the advent of warnings concerning the environment and global warming, numerous international conferences have been held resulting in treaties wherein sovereign nations have avowed a goal to reduce carbon emissions from industry and government. To that end, many governments have and will derive standards requiring business and individuals and industries to reduce their greenhouse gas emissions with the goal of a worldwide reduction of greenhouse gases emitted into the atmosphere.

Greenhouse gases (GHG), such as carbon dioxide (CO₂) and methane (CH₄), are emitted from several different sources, both natural and artificial; however, the two sources with the most emphasis are the agricultural and the fossil fuel industry. Within agriculture, ruminants and in particular cattle are the major contributors to the anthropogenic methane formation, not only via manure but in particular by eructation of methane produced during rumen fermentation processes.

Rumen methane inhibitors such as e.g. 3-nitrooxypropanol, nitrate and bromoform but also certain seaweed have been reported to significantly reduce the methane production during rumen fermentation and thus can significantly contribute to reduce the methane emissions of ruminants by exhalation.

Emissions Reduction Currency Systems (ERCS) are a component of national and international attempts to mitigate the overall growth in concentrations of greenhouse gases. With ERCS it is possible to establish positive economic reward systems for reductions in greenhouse gas emissions, e.g. through the emission of coupons, tokens, reward points, or complementary currencies which in turn can motivate an individual or a group of individuals to start to invest into greenhouse gas reduction projects. An already established system is, for example a 'carbon credit', a tradable certificate respectively permit representing the right to emit one ton of carbon dioxide or the equivalent amount of a different greenhouse gas (based on a reduction thereof elsewhere). The value of traded global markets for carbon dioxide allowances hit record 144 billion euros in 2018 while continuing to grow.

However, in order to successfully being able to participate in any such trading system, the GHG emission reduction must be readily measurable and reliably quantifiable by simple and economic means. Therefore, it is important to be able to quantify the methane emission reduction effected by the supplementation of a methane inhibitor in ruminants with a high accuracy while avoiding background noise (e.g. by methane emissions from manure) e.g. in a loose house environment.

Current methods for measuring methane emission reduction in ruminants by the supplementation of a methane inhibitor are either not yet selective enough or require sophisticated measurement methods and/ or devices such as GreenFeeds, metabolic respiration chambers or inert gas marker methods, which are not suitable to be used economically on large scale.

LOPES J C ET AL: "Effect of 3-nitrooxypropanol on methane and hydrogen emissions, methane isotopic signature, and ruminal fermentation in dairy cows", JOURNAL OF DAIRY SCIENCE, vol. 99, no. 7, 13 April 2016 (2016-04-13), pages 5335-5344 discloses the use of a methane inhibitor (3-nitrooxypropanol) as an oral supplementation to ruminants, and monitoring emissions of CH4, CO2 and H2 from said ruminants after that.

Thus, there is an ongoing need for an efficient, quantitative and reliable method to monitor and assess the reduction in methane emission affected by the supplementation of a methane inhibitor.

Surprisingly, it has now been found that the amount of volatile organic compounds (VOCs) in the exhaled air of ruminants supplemented with a methane inhibitor is significantly increased and correlates with the methane emission reduction and can thus be used to monitor and/or quantify the methane emission reduction resulting from said supplementation using simple detection devices.

Thus, in a first embodiment, the present invention relates to the use of a methane inhibitor to increase the volatile organic compound (VOC) emissions of ruminants after oral supplementation thereof and optionally appreciating the effect.

In a second embodiment, the present invention relates to a method to increase the volatile organic compound emissions of ruminants, said method comprising the supplementation of a methane inhibitor to said ruminant and monitoring said VOC emission increase.

The increase in the volatile organic compound emissions (in the exhaled air) of the ruminant(s) can be used to monitor the supplementation of the methane inhibitor and/ or for the quantification of the methane emission reduction affected by the supplementation of the methane inhibitor.

The term 'volatile organic compound (VOC)' as used herein is well known to a person skilled in the art and refers to organic chemicals that have a high vapor pressure at ordinary room temperature and a low water solubility. Their high vapor pressure results from a low boiling point, which causes large numbers of molecules to evaporate or sublimate from the liquid or solid form of the compound and enter the surrounding air, a trait known as volatility. It is well understood to a person skilled in the art, that organic acids such as fatty acids are not volatile organic compounds as they readily dissolve in water and/ or have a high boiling point. According to common global standards VOC exhibit an initial boiling point less than or equal to 250 °C (482 °F) measured at a standard atmospheric pressure of 101.3 kPa. VOC's are also commonly defined by their Henry law constant (H) being in the range of 5*10⁻³-10⁻⁵ atm*m³/mol (Henry's law constants (H) defining the volatility from liquid to air at temperatures of 20-25°C).

It is also well understood, that the methane inhibitor does not lead to an increase but to a decrease in methane emissions and thus the term VOC as used herein does not encompass methane.

The term 'to increase the volatile organic compound (VOC) emissions' refers to an overall increased amount of VOC's emitted by the ruminant compared to control animal(s), which is measurable e.g. with devices or methods as outlined herein. Said increase can be identified by using certain marker VOC's but also by measuring the overall VOC's detectable with suitable sensors as outlined herein.

In all embodiments of the present invention, said VOC's preferably exhibit a boiling point of less than 200°C, more preferably of less than 150°C, most preferably of less than 130°C at atmospheric pressure (i.e. 101.3 kPa).

In all embodiments of the present invention, said VOC's preferably exhibit a water solubility of less than 3 g/l, preferably less than 2.5 g/l, most preferably less than 2 g/l at 20°C.

In all embodiments of the present invention, saidVOC's preferably exhibit a molecular weight of less than 150 g/mol, more preferably of less than 140 g/mol, most preferably of less than 125 g/mol.

Particularly preferred VOC's according to the present invention exhibit
1. a molecular weight of less than 125 g/mol,
2. a boiling point of less than 130°C at atmospheric pressure, and
3. a water solubility of less than 2 g/l at 20°C.

Exemplary VOCs which abundance is increased in the exhaled air of the ruminants by the supplementation of the methane inhibitor include propane, propene, isobutane, butane, 1,3-pentadiene, benzene, hexane, heptane and octane without being limited thereto. It is well understood, that said VOC's can e.g. be used as tracer VOC's for all uses and methods as used herein.

Thus, preferably, in all embodiments of the present invention the VOC emissions comprise hydrocarbons with two or more carbon atoms, preferably selected from the group consisting of propane, propene, isobutane, butane, 1,3-pentadiene, benzene, hexane, heptane and octane.

Preferably, the VOC's measured according to the present invention encompass at least two or more, preferably all of propane, propene, isobutane, butane, 1,3-pentadiene, benzene, hexane, heptane and octane.

Even more preferably, the VOC's measured according to the present invention consist essentially of propane, propene, isobutane, butane, 1,3-pentadiene, benzene, hexane, heptane and octane.

It is well understood in all embodiments of the present invention that next to the VOC increase, the methane emissions are concomitantly reduced, preferably by at least 10 %, more preferably by at least 20%, most preferably by at least 30%, compared to an untreated control (baseline) through the supplementation of the methane inhibitor.

Accordingly, in all embodiments of the present invention, the methane inhibitor is administered to the ruminant in an amount which results in a methane reduction, preferably of at least 10 %, more preferably of at least 20 %, most preferably of at least 30 %, such as even of at least 40 %, when compared to control, i.e. to ruminants not supplemented with the methane inhibitor.

The increase in the VOC can be measured by various techniques known in the art.

In one embodiment, the increase in the VOCs is measured by capturing the breath (exhaled air) of a ruminant with a commercial horse nebuliser system connected to a thermal desorption sorbent sampling tube for VOCs such as air toxic analyser tubes (part no.C2-AXXX-5270) or Bio-monitoring tubes (part no. C2-CXXX-5149) available from Markes International Ltd. Preferably, the breath is collected for 5 minutes at a flow rate of 200 ml/min, giving a total capture volume of 1L. Alternatively, a fast-flow pump kit for sampling VOCs (e.g. available as MTS-32 tube capture systems fitted with an Acti-VOC low-flow rate pump system and a battery pack system from Markes International Ltd.) can be used. The advantage of the latter system is, that it allows the automated capture of air volatiles over an extended period of time, without input from an operator from a group of ruminants as it can easily be installed in ruminant farm housing setting. The tubes used for capturing the VOCs can be analyzed by methods well known in the art such as GC-TOF-MS (gas chromatograph-time of flight-mass spectrometer), e.g. available from Markes International as Markes BenchTOF-HD with a Thermo Trace 1300 GC unit, optionally fitted with an automated thermal desorption unit (Markes Unity xr and TD100 systems).

In a particular embodiment, masses from 25 to 250 atu (atomic mass units) are monitored. Quantification of the captured VOCs can be performed by integration of the peaks. This method does not only allow the quantification of the VOC increase but also allows to detect changes in the VOC composition after the supplementation of the methane inhibitor.

In another and preferred embodiment, however the VOC emission increase according to the present invention is monitored using suitable VOC sensors as said sensors were found to also reliably monitor said increase in a ruminant farm house setting thus allowing real time assessment by using low cost, small sensors. VOC sensors generally provide an aggregate level of organic compounds which combines all the organic compounds present in the air of the ruminant farm house setting.

Therefore in a particular advantageous embodiment the present invention also relates to a method to monitor and/ or quantify the supplementation of a methane inhibitor to one or more ruminants in a ruminant farm house setting, said method comprising the steps of:
a) providing at least one VOC sensor in said farm house setting,
b) determining the baseline VOC emission of said farm house setting over a time period (t),
c) determining the VOC emission in said farm house setting over the same time period (t) while supplementing the methane inhibitor to the one or more ruminants, followed by
d) determining the VOC emission increase during said time period (t) over the baseline VOC emission.

Exemplary VOC sensors include ampirometric sensors (or electrochemical), chemoresistive sensors employing metal-oxide materials and optical techniques based on non-dispersive infra-red technology (NDIR).

The VOC sensor can e.g. be an ion detector using photoionization of the molecules in an air sample collected in the ruminant farm house setting of interest or an electrochemical MOS on-chip sensor using oxidation-reduction reactions with gas(es) present in ambient air, to assess VOC concentration level in the ruminant farm house setting.

Particularly suitable sensors to monitor/ measure the (total) VOC increase according to the present invention are ethylene oxide sensors, e.g. commercially available as Alphasense ETO-A1 as well as digital VOC sensors, preferably the ones using a Pd dopant. Exemplary VOC sensors are e.g. commercially available as AMS iAQ-Core C from AMS, ScioSence CCS811, or Sensirion SGP30 from Sensirion. Most preferred in all embodiments according to the present invention is the use of an ethylene oxide sensor.

The amount of the VOCs measured can be indicated either in ppm or in output voltage. Both output data can be used for the purposes of the present invention.

Preferably, in all embodiments of the present invention the VOC sensor is incorporated into a monitoring unit. Such monitoring units are well known to a person skilled in the art and generally encompass the sensor, a cover with ventilation holes, a fan mounted back panel, a circuit board, a display to show current values from the sensors (e.g. a TFT display), a USB (mini) connector, a micro-controller (e.g. ESPRESSIF ESP32), an on/off button and a power supply connector. Said units can be powered e.g. by using external USB-power packs. Said monitoring units can comprise a SD card to record values. Alternatively or in addition the monitoring unit can be directly connected to a computing device or send the VOC concentration information to a remote computing device such as a smartphone. Preferably, the gained data is stored on the computing device and can even be used as input for a machine learning algorithm.

The present invention also provides uses and methods according to the present invention wherein the concentration of the VOCs are measured with a VOC sensor and wherein the VOC concentration data is stored in a memory of a computing unit. Optionally the VOC concentration data can be sent from the computing unit to a remote entity such as a computer or a smartphone e.g. over a wireless link. Preferably the transmission to the remote entity occurs at regular intervals.

The VOC emission increase for a defined time period (t) can be calculated as difference of the baseline VOC emission over said period of time before supplementation and the emissions over the same time period during the supplementation. It is well understood, that the farm house setting has to be calibrated once for a defined period of time. Afterwards, the emission increase can be assessed repeatedly for said time period (t). Suitable time periods (t) encompass e.g. 4 h, 8 h, 12 h, 15 h, 20 h, 24 h, 30 h or 40 h without being limited thereto.

Preferably the time period is selected such, that the methane inhibitor is supplemented at least once, preferably at least twice, most preferably at least 3 times to the ruminants during said time period.

It is well understood that the measurement of the VOC emission and the quantification can be fully automatized using appropriate mathematical models.

In the context of the present invention, the term 'ruminant farm house setting' is to be understood in a broad manner and includes any facility which is housing ruminants, preferably cattle, most preferably dairy cows. Preferably, the farm house setting houses at least 5, preferably at least 10, more preferably at least 50, even more preferably at least 100, most preferably at least 150 ruminants.

The correlation of the VOC emission increase with the reduction in methane emission can be performed by calibrating the methane reduction with said VOC increase beforehand by measuring both emissions using a reliable method. The VOC emissions can be measured as outlined herein, while the methane emission can be measured according to standard methods in the art such as using e.g. a GreenFeed system or a metabolic respiration chamber.

The VOC emission increase (over the baseline) can furthermore be correlated to the amount of methane inhibitor supplemented.

The use and methods as outlined herein allow for an automized and validated determination of the methane reduction and/ or of CO₂ equivalents resulting from the supplementation of a methane inhibitor.

The term 'methane inhibitor' as used herein relates to all compounds suitable to reduce the methane emissions in ruminants (i.e. rumen methane inhibitors). Suitable methane inhibitors according to the present invention include garlic extracts, allicin, 3-nitrooxypropanol, bromoform, chloroform, nitrate, nitroethane, lauric acid, lauricidin, marine algae as well as red algae in particular of the genus Asparagopsis (e.g. Asparagopsis taxiformis or Asparagopsis armata) without being limited thereto. Preferred methane inhibitors in all embodiments of the present invention are garlic extracts, allicin, red algae of the genus Asparagopsis, preferably Asparagopsis taxiformis or Asparagopsis armata and 3-nitrooxypropanol as well as mixtures thereof. Most preferred in all embodiments of the present invention is the methane inhibitor 3-nitrooxypropanol, which is particularly effective.

3-Nitrooxypropanol (also referred to Propanediol mononitrate, 1,3-propanediol mononitrate, or PDMN [CAS No: 100502-66-7]) is a known compound which can e.g. be manufactured as outlined in WO2004043898 or WO2012084629 and is available at DSM Nutritional Products Ltd under the tradename Bovaer^{®}, e.g. as a 10% form on SiO₂.

The methane inhibitor may be supplemented as such or in the form of a composition comprising said methane inhibitor, which composition may be formulated in any suitable form, including a powder, a granule, a pellet, a solution, or a suspension.

Said composition can be a dry, free- flowing powder (powderous formulation) suitable for direct inclusion into a commercially available feed or as a supplement to a total mixed ration or diet. The powderous formulation may be mixed with either solid or liquid feed or with water. In another embodiment, the composition can be formed into pellets or tablets.

When the methane inhibitor is 3-nitrooxypropanol, then the composition preferably is a powderous formulation comprising 3-nitrooxypropanol and a carrier material. Suitable carrier includes any carrier well known in the food and feed industry such as silicone dioxide without being limited thereto.

If the composition is a powderous formulation comprising a methane inhibitor such as preferably 3-nitrooxypropanol and a carrier material, the methane inhibitor is usually sprayed onto or admixed with the carrier material by standard methods in the art, e.g. by using solvent suitable for the preparation of food or feed products such as e.g. dichloromethane followed by evaporation of the organic solvent.

Alternatively, the methane inhibitor such as preferably 3-nitrooxypropanol can be diluted in a suitable edible oil before being sprayed onto or admixed with the carrier material. The powderous formulation may in addition contain usual additives used in the preparation of powderous formulations for feed application.

The amount of the methane inhibitor in the composition according to the present invention, in particular in a powderous formulation is preferably selected in the range of 1 to 20 wt.-%, preferably in the range of 2 to 15 wt.-%, most preferably in the range of 4 to 12 wt.-%, based on the total weight of the composition.

Preferably, in all embodiments of the present invention, the amount of the methane inhibitor administered to the ruminants is selected in the range from 0.01 to 100 g methane inhibitor/ animal/ day, more preferably in the range from 0.01 to 50 g methane inhibitor/ animal/ day, most preferably in the range from 0.01 to 25 g methane inhibitor/ animal/ day, such as in the range from 0.01 to 10 g methane inhibitor/ animal/ day, 0.01 to 5 g methane inhibitor/ animal/ day or 0.01 to 2.5 g methane/ animal/ day.

Preferably, in all embodiments of the present invention, the amount of 3-nitrooxypropanol administered to the ruminants is selected in the range from 0.05 to 5 g PDMN/ animal/ day, more preferably in the range from 0.1 to 4 g PDMN/ animal/ day, most preferably in the range from 0.25 to 3 g PDMN/ animal/ day. Further suitable effective amounts are selected in the range from 0.5 to 3 g PDMN/ animal/ day or from 1 to 3 g PDMN/ animal/ day.

The methane inhibitor respectively the composition comprising the methane inhibitor according to the present invention such as in particular the powderous formulation is preferably administered admixed with the animal's feed, such as for example forage (grass, legumes, silage), hay, grass, grain as well as soy without being limited thereto.

Preferably, the daily amount of the methane inhibitor is supplemented admixed with the feed and supplemented according to the regular feeding regimen of the ruminants. Advantageous, in all embodiments of the present invention, the amount of the methane inhibitor in said feed is selected in the range from 1 mg to about 25 g per kg dry matter feed, preferably from about 1 mg to about 10 g per kg dry matter feed, more preferably from about 10 mg to about 1 g per Kg dry matter feed, most preferably from 20 mg to 500 mg per Kg of dry matter feed, such as from about 20 mg to 250 mg per Kg of dry matter feed, even more preferably from 20 mg to 200 mg per dry matter kg feed such as in the range from 20 mg to 150 mg per kg dry matter feed or 50 mg to 100 mg per kg dry matter feed.

Daily dry matter intake for cattle is generally in the range of 1 to 3.5 % of dry matter per kg live weight. The amount of dry matter intake (DMI) for dairy cows is, for example, about 2-3 % dry matter per kg live weight.

In the present context, a ruminant is a mammal of the order Artiodactyla that digests plant-based food by initially softening it within the animal's first stomach, known as the rumen, then regurgitating the semi-digested mass, now known as cud, and chewing it again. The process of again chewing the cud to further break down plant matter and stimulate digestion is called "ruminating".

Ruminants according to the present invention include cattle, goats, sheep, giraffes, bison, yaks, buffalo, deer, camels, alpacas, llamas, wildebeest, antelope, pronghorn, and nilgai.

In all embodiments of the present invention, buffalos, domestic cattle, sheep and goats are the more preferred species. For the present purposes, the most preferred species are domestic cattle. The term *'cattle'* as used herein includes all races of domestic cattle, and all production kinds of cattle, in particular dairy cows and beef cattle.

A particularly preferred group of ruminants in all embodiments according to the present invention are dairy cows, in particular lactating dairy cows or beef cattle.

The present invention is further described by the following examples which should not be construed as limiting the scope of the invention.

### Example

### Methane inhibitor supplementation

All cows were fed fresh grass to which over 24h six times (i.e. at t = 0, 4, 8, 12, 16, 24) a small amount of a supplemental feed comprising 250 mg ground corn and, in the treatment group, 0.267g of 3-nitrooxypropanol was added (1 treatment and 2 control groups).

### Real time VOC measurement

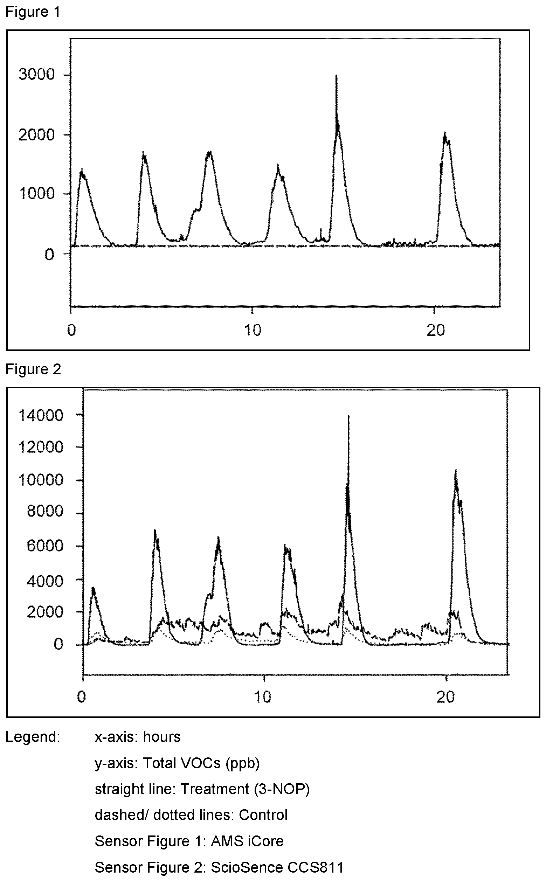

A full custom PCB was designed to house and measure the responses from the sensors. Sensors used encompassed an AMS iAQ-Core C (AMS iCore) and a ScioSence CCS811 sensor. The system was fitted with an ESPRESSIF ESP32 micro-controller, SD card to record values and a large display to show current values from the sensors. The unit was fitted into a small white box, which was machined to allow to air pass through the front of the instrument to an outlet port on the side of the instrument. A fan has been added to the unit to assist in pulling air through the unit and is on all the time. The unit was powered using external USB power packs (from Power Banks). The unit was placed in a metabolic respiration chamber together with a cow and VOCs emitted were measured by the respective sensors (AMS iCore (Figure 1); ScioSence CCS811 (Figure 2)).
eight hydrocarbons

VOC's identified to exhibit a good correlation with the overall VOC increase over a broad range of animals (analysed via GC-TOF-MS) were propane, propene, isobutane, butane, 1,3-pentadiene, benzene, hexane, heptane and octane.

As can be retrieved from Figure 1 and 2, the supplementation of the methane inhibitor effected a significant increase in the total amount of VOCs compared to the control group.

## Claims

1. Use of a methane inhibitor to increase volatile organic compound (VOC) emissions of one or more ruminants after oral supplementation thereof, wherein the increased VOC emissions are used to monitor the supplementation of the methane inhibitor to the ruminants.

2. The use according to claim 1, wherein the increased VOC emissions are used to quantify the methane emission reduction affected by the supplementation of the methane inhibitor to the ruminants.

3. The use according to claim 1 or 2, wherein the volatile organic compounds comprise at least two hydrocarbons with two or more carbon atoms, preferably selected from the group consisting of propane, propene, isobutane, butane, 1,3-pentadiene, benzene, hexane, heptane and octane.

4. The use according to anyone of the preceding claims, wherein the ruminants are dairy cows or beef cattle, preferably dairy cows, most preferably lactating dairy cows.

5. The use according to anyone of the preceding claims, wherein the methane inhibitor is 3-nitrooxypropanol.

6. The use according to anyone of the preceding claims, wherein the increase in volatile organic compound emissions is measured with an ethylene oxide sensor and/ or a digital VOC sensor.

7. The use according to claim 5, wherein the digital VOC sensor is one using a Pd dopant.

8. The use according to claim 6 or 7, wherein the increase in the volatile organic compound emissions is measured in a ruminant farm-house setting.

9. The use according to anyone of the preceding claims for an automized and validated determination of the methane reduction affected by the supplementation of the methane inhibitor.

10. A method to increase the volatile organic compound (VOC) emissions of one or more ruminants, said method comprising the supplementation of a methane inhibitor to said ruminants and monitoring said VOC emission increase, wherein the volatile organic compounds comprise at least two hydrocarbons with two or more carbon atoms, preferably selected from the group consisting of propane, propene, isobutane, butane, 1,3-pentadiene, benzene, hexane, heptane and octane.

11. The method according to claim 10, wherein the method is used in a ruminant farm house setting.

12. The method according to claim 11, wherein the ruminant farm house setting houses at least 5, preferably at least 10, more preferably at least 50, even more preferably at least 100, most preferably at least 150 ruminants.

13. The method according to claim 11 or 12, wherein said method comprises the steps of:
a. providing a VOC sensor in the farm house setting,
b. determining the baseline VOC emission of said farm house setting over a defined time period (t),
c. determining the VOC content in said farm house setting over the same time period (t) during which time period the methane inhibitor is supplemented to the ruminants, followed by
d. determining the VOC emission increase over baseline.

14. The method according to anyone of claims 10 to 13, wherein the method is used to quantify the reduction of methane emissions resulting from the supplementation of the methane inhibitor to the ruminants.

15. The method according to anyone of claims 10 to 14, wherein the VOC sensor is incorporated into a monitoring unit connected to a remote computing device.

## Patentansprüche

1. Verwendung eines Methanhemmers zur Erhöhung der Emissionen flüchtiger organischer Verbindungen (VOC) eines oder mehrerer Wiederkäuer nach oraler Supplementierung davon, wobei die Supplementierung des Methanhemmers zu den Wiederkäuern anhand der erhöhten VOC-Emissionen überwacht wird.

2. Verwendung nach Anspruch 1, wobei die durch die Supplementierung des Methaninhibitors zu den Wiederkäuern beeinflusste Methanemissionsreduktion anhand der erhöhten VOC-Emissionen quantifiziert wird.

3. Verwendung nach Anspruch 1 oder 2, wobei die flüchtigen organischen Verbindungen mindestens zwei Kohlenwasserstoffe mit zwei oder mehr Kohlenstoffatomen umfassen, vorzugsweise ausgewählt aus der aus Propan, Propen, Isobutan, Butan, 1,3-Pentadien, Benzol, Hexan, Heptan und Octan bestehenden Gruppe.

4. Verwendung nach einem der vorhergehenden Ansprüche, wobei es sich bei den Wiederkäuern um Milchkühe oder Mastrinder, vorzugsweise Milchkühe, am meisten bevorzugt laktierende Milchkühe, handelt.

5. Verwendung nach einem der vorhergehenden Ansprüche, wobei der Methaninhibitor 3-Nitrooxypropanol ist.

6. Verwendung nach einem der vorhergehenden Ansprüche, wobei der Anstieg der Emissionen flüchtiger organischer Verbindungen mit einem Ethylenoxidsensor und/oder einem digitalen VOC-Sensor gemessen wird.

7. Verwendung nach Anspruch 5, wobei der digitale VOC-Sensor einer ist, der einen PD-Dotierstoff verwendet.

8. Verwendung nach Anspruch 6 oder 7, wobei der Anstieg der Emissionen flüchtiger organischer Verbindungen in einem Wiederkäuerlandwirtschaftsbetrieb gemessen wird.

9. Verwendung nach einem der vorhergehenden Ansprüche für eine automatisierte und validierte Bestimmung der durch die Supplementierung des Methaninhibitors beeinflussten Methanreduktion.

10. Verfahren zur Erhöhung der Emissionen flüchtiger organischer Verbindungen (VOC) eines oder mehrerer Wiederkäuer, wobei das Verfahren die Supplementierung eines Methaninhibitors zu den Wiederkäuern und die Überwachung der Erhöhung der VOC-Emission umfasst, wobei die flüchtigen organischen Verbindungen mindestens zwei Kohlenwasserstoffe mit zwei oder mehr Kohlenstoffatomen umfassen, vorzugsweise ausgewählt aus der aus Propan, Propen, Isobutan, Butan, 1,3-Pentadien, Benzol, Hexan, Heptan und Octan bestehenden Gruppe.

11. Verfahren nach Anspruch 10, wobei das Verfahren in einem Wiederkäuerlandwirtschaftsbetrieb angewendet wird.

12. Verfahren nach Anspruch 11, wobei in dem Wiederkäuerlandwirtschaftsbetrieb mindestens 5, vorzugsweise mindestens 10, besonders bevorzugt mindestens 50, noch mehr bevorzugt mindestens 100, am meisten bevorzugt mindestens 150 Wiederkäuer untergebracht sind.

13. Verfahren nach Anspruch 11 oder 12, wobei das Verfahren die folgenden Schritte umfasst:
a. die Bereitstellung eines VOC-Sensors in dem Landwirtschaftsbetrieb,
b. die Bestimmung der VOC-Basislinienemission des Landwirtschaftsbetrieb über einen definierten Zeitraum (t),
c. die Bestimmung des VOC-Gehalts in dem Landwirtschaftsbetrieb über den gleichen Zeitraum (t), in dem der Methaninhibitor zu den Wiederkäuern supplementiert wird, gefolgt von
d. der Bestimmung des Anstiegs der VOC-Emission gegenüber dem Ausgangswert.

14. Verfahren nach einem der Ansprüche 10 bis 13, wobei mit dem Verfahren die Reduktion von Methanemissionen, die sich aus der Supplementierung des Methaninhibitors zu den Wiederkäuern ergeben, quantifiziert wird.

15. Verfahren nach einem der Ansprüche 10 bis 14, wobei der VOC-Sensor in eine Überwachungseinheit integriert ist, die mit einem entfernten Computer verbunden ist.

## Revendications

1. Utilisation d'un inhibiteur de méthane pour augmenter les émissions de composés organiques volatils (COV) d'un ou plusieurs ruminants après supplémentation orale avec celui-ci, dans laquelle les émissions accrues de COV sont utilisées pour surveiller la supplémentation avec l'inhibiteur de méthane des ruminants.

2. Utilisation selon la revendication 1, dans laquelle les émissions accrues de COV sont utilisées pour quantifier la réduction des émissions de méthane affectée par la supplémentation avec l'inhibiteur de méthane des ruminants.

3. Utilisation selon la revendication 1 ou 2, dans laquelle les composés organiques volatils comprennent au moins deux hydrocarbures avec au moins deux atomes de carbone, de préférence choisis dans le groupe constitué par le propane, le propène, l'isobutane, le butane, le 1,3-pentadiène, le benzène, l'hexane, l'heptane et l'octane.

4. Utilisation selon l'une quelconque des revendications précédentes, dans laquelle les ruminants sont des vaches laitières ou des bovins de boucherie, de préférence des vaches laitières, idéalement des vaches laitières en lactation.

5. Utilisation selon l'une quelconque des revendications précédentes, dans laquelle l'inhibiteur de méthane est le 3-nitrooxypropanol.

6. Utilisation selon l'une quelconque des revendications précédentes, dans laquelle l'augmentation des émissions de composés organiques volatils est mesurée avec un capteur d'oxyde d'éthylène et/ou un capteur de COV numérique.

7. Utilisation selon la revendication 5, dans laquelle le capteur de COV numérique en est un utilisant un dopant au Pd.

8. Utilisation selon la revendication 6 ou 7, dans laquelle l'augmentation des émissions de composés organiques volatils est mesurée dans l'environnement d'une ferme de ruminants.

9. Utilisation selon l'une quelconque des revendications précédentes pour une détermination automatisée et validée de la réduction de méthane affectée par la supplémentation avec l'inhibiteur de méthane.

10. Procédé d'augmentation des émissions de composés organiques volatils (COV) d'un ou plusieurs ruminants, ledit procédé comprenant la supplémentation avec un inhibiteur de méthane desdits ruminants et la surveillance de ladite augmentation des émissions de COV, dans lequel les composés organiques volatils comprennent au moins deux hydrocarbures avec au moins deux atomes de carbone, de préférence choisis dans le groupe constitué par le propane, le propène, l'isobutane, le butane, le 1,3-pentadiène, le benzène, l'hexane, l'heptane et l'octane.

11. Procédé selon la revendication 10, le procédé étant utilisé dans l'environnement d'une ferme de ruminants.

12. Procédé selon la revendication 11, dans lequel l'environnement de la ferme de ruminants abrite au moins 5, de préférence au moins 10, mieux au moins 50, mieux encore au moins 100, idéalement au moins 150 ruminants.

13. Procédé selon la revendication 11 ou 12, ledit procédé comprenant les étapes suivantes :
a. mise en place d'un capteur de COV dans l'environnement de la ferme,
b. détermination des émissions de COV de référence dudit environnement de la ferme sur un laps de temps défini (t),
c. détermination de la teneur en COV dans ledit environnement de la ferme sur le même laps de temps (t), laps de temps pendant lequel les ruminants sont supplémentés avec l'inhibiteur de méthane, puis
d. détermination de l'augmentation des émissions de COV par rapport au niveau de référence.

14. Procédé selon l'une quelconque des revendications 10 à 13, le procédé étant utilisé pour quantifier la réduction des émissions de méthane résultant de la supplémentation avec l'inhibiteur de méthane des ruminants.

15. Procédé selon l'une quelconque des revendications 10 à 14, dans lequel le capteur de COV est incorporé dans une unité de surveillance connectée à un dispositif informatique distant.
